(19) Europäisches Patentamt
European Patent Office
Office européen des brevets

(11) Publication number: **0 317 627 B1**

# (12) EUROPEAN PATENT SPECIFICATION

(45) Date of publication of patent specification: **06.09.95** (51) Int. Cl.$^6$: **C07K 14/475**

(21) Application number: **88907473.8**

(22) Date of filing: **26.05.88**

(86) International application number:
**PCT/US88/01767**

(87) International publication number:
**WO 88/09378 (01.12.88 88/26)**

## (54) ONCOGENE-ENCODED POLYPEPTIDE HAVING GROWTH FACTOR ACTIVITY AND METHODS OF PRODUCTION AND USE THEREOF.

(30) Priority: **29.05.87 US 56137**

(43) Date of publication of application:
**31.05.89 Bulletin 89/22**

(45) Publication of the grant of the patent:
**06.09.95 Bulletin 95/36**

(84) Designated Contracting States:
**AT BE CH DE FR GB IT LI LU NL SE**

(56) References cited:

**Nature, (London, England), Volume 300, issued 9 December 1982, (PULCIANI et al.), "Oncogenes in Solid Human Tumours", see page 539.**

(73) Proprietor: **THE TRUSTEES OF COLUMBIA UNIVERSITY IN THE CITY OF NEW YORK**
**West 116th Street and Broadway**
**New York,**
**New York 10027 (US)**

(72) Inventor: **GOLDFARB, Mitchell**
**327 Taft Road**
**River Edge, NJ 07661 (US)**
Inventor: **ZHAN, Xi**
**60 Haven Avenue**
**New York, NY 10032 (US)**

(74) Representative: **Henkel, Feiler, Hänzel & Partner**
**Möhlstrasse 37**
**D-81675 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Nature, (London, England), Volume 303, issued 2 June 1983, (HALL et al.), "Identification of Transforming Gene in two Human Sarcoma Cell Lines as a New Member of the Ras Gene Family Located on Chromosome 1", see pages 396 and 397.

Nature, (London, England), Volume 326, issued 30 April 1987, (DICKSON et al.) "Potential Oncogene Product Related to Growth Factors", see page 833

Nature, (London, England), Volume 316, issued 18 July 1985, (EVA et al.), "Isolation of a New Oncogene from a Diffuse B-Cell Lymphoma", see page 273.

Science, (Washington, D.C., U.S.A.), Volume 233, issued 1 August 1986, (ABRAHAM et al.), "Nucleotide Sequence of a Bovine Clone Encoding the Angiogenic Protein, Basic Fibroblast Growth Factor", see page 545.

Science, (Washington, D.C. U.S.A.), Volume 230, issued 20 December 1985, (GIMENEZ-GALLEGO) "Brain-Derived Acidic Firboblast Growth Factor: CompleteAmino Acid Sequence and Homologies", see page 1385.

The EMBO Journal, (Oxford, England) Volume 5, issue May 1986, (MOORE et al)"Sequence, Topagraphy, and Protein Coding Potential of Int-2: a Putative Oncogene Activated by Mouse Mammary Tumour Virus", see page 729.

Cell, (Cambridge, Massachusetts, USA) Volume 50, issued August 1987, (BOVI et al.), "An Oncogene Isolated by Transfection of Kaposi's Sarcoma DNA Encodes a Growth Factor That Is a Member of the FGF Family", s.p. 729

Molecular and Cellular Biology, (Washington, D.C., U.S.A.), Volume 6, issued October 1986, (VOGT et al.), "Coinfection with Viruses Carrying the v-Ha-Rasand v-Myc Oncogenes Leads to Growth Factor Independence by an Indirect Mechanism", see page 3545.

Molecular and Cellular Biology, (Washington, D.C. U.S.A.), Volume 6, issued October 1986, (ZHAN et al.), "Growth Factor Requirements of Oncogene-Transformed NIH3T3 and Balb/c 3T3 Cells Culture in Defined Media", see page 3541.

Proceedings of the National Academy of Sciences, (Washington, D.C., USA) Volume 82, issued September 1985, (FUKUI et al.), "Detection of Raf-Related and two other Transforming DNA Sequences in Human Tumors Maintained in Nude Mile", see page 5854.

ONCOGENE, vol. 1, 1987, pp. 369-376, The Macmillan Press Ltd; X. ZHAN et al.: "Human oncogenes detected by a defined medium culture assay"

MOL. CELL. BIOL., vol. 8, Augsut 1988, pp. 3487-3495, American Society of Microbiology; X. ZHAN et al.: "The human FGF-5 oncogene encodes a novel protein related to fibroblast growth factors"

## Description

### Background of the Invention

Throughout this application various publications are referenced by numbers within parentheses. Full citations for these publications may be found at the end of the specification immediately preceding the claims.

DNA-mediated gene transfer (DNA transfection) into NIH 3T3 murine fibroblast cells has been a fruitful means for detecting oncogenes in mammalian cellular DNA. The most extensively used transformation assay is the NIH 3T3 focus assay, in which transformed 3T3 cells are detected by their growth into dense foci (1). The focus assay has detected activated oncogenes in the genomes of many tumors and tumor-derived cell lines (2-6). The detected oncogenes are usually members of the ras gene family (5-11), although other genes have been characterized as well (5, 12-15). The alternative oncogene assay monitors tumor formation in immunodeficient mice following injection with transfected NIH 3T3 cells. The oncogenes met (16) and mas (17) were discovered by this method.

This invention discloses the use of a new transformation assay to detect a novel oncogene in DNA from human tumor-derived cell lines. The amino acid sequence encoded by the cDNA of this oncogene shows substantial homology to the previously characterized fibroblast growth factors, bFGF and aFGF (40,41), as well as to the amino acid sequence of two recently characterized oncogenes, int-2 and hst (42,43). For this reason applicants have designated this oncogene FGF-3 and continue this designation in the subject application. However, FGF-3 may alternatively be designated FGF-5 in future publications by the applicants.

### Summary of the Invention

This invention provides a purified polypeptide having growth factor activity and the amino acid sequence shown in Figure 5 for either ORF-2 or ORF-1. This invention also provides a purified nucleic acid molecule encoding the polypeptides.

This invention further provides a vector which comprises the nucleic acid molecule encoding the polypeptide as well as a host vector system for producing the polypeptide.

This invention also provides a pharmaceutical composition comprising an effective amount of the polypeptide shown in Figure 5 for ORF-2 and pharmaceutically acceptable carrier. The invention further provides a method of stimulating the proliferation of mesodermal cells, capillary growth and promoting tissue repair by administering an effective amount of the pharmaceutical composition.

This invention also provides an isolated fragment of total human genomic DNA comprising the oncogene encoding the amino acid sequence shown in Fig. 5 for ORF-2. Additionally, this invention provides a molecule useful as a probe for detecting the oncogene.

This invention still further provides a reagent comprising an antibody capable of specifically forming a complex with the polypeptide of this invention and said reagent for diagnosing a neoplastic condition associated with the presence of an activated oncogene.

### Brief Description of the Figures

Figure 1 shows a protocol for detecting oncogenes by defined medium culture. The steps include: 1) Transfect each 100mm plate with 30 micrograms tumor DNA + 1 microgram pLTRneo. 8 hr. 2) Refeed with DME + serum.O/N. 3) Trypsinize. Split 1:2. 4) DME + serum + G418. 12 Days. 5) Trypsinize. 6) Plate $10^5$ cells on fibronectin coated $35^{mm}$ dish. Refeed and maintain in defined medium w/o PDGF/FGF. 7) 8 days.

Figure 2 shows human Alu repeat sequences in transformants derived from VMCUB2 tumor DNA transfections. EcoRI-digested DNAs from primary and secondary transformants of VMCUB2 were analyzed by filter blot hybridization with pSP6-Alu 32P-RNA. DNA from primary transformant VMCUB2-2 (lane a) and its secondary transformants (b-e), primary transformant VMCUB2-1 (f) and its secondary transformants (g-i). Arrows denote size markers (in kbp).

Figure 3 shows the physical map of the FGF-3 oncogene. The inserts of three overlapping genomic clones are shown. Kilobase pair lengths (in kbp) of EcoRI (R) restriction fragments are indicated. Fragments containing Alu repeats are indicated with vertical hash-marks, fragments homologous to the oncogene cDNA clone are indicated as solid boxes, and the 2.8 kbp fragment derived from pLTRneo is marked with diagonal stripes.

Figure 4 shows the hybridization of RNAs to the FGF-3 oncogene cDNA probe. Cytoplasmic RNAs (10 micrograms) from secondary transformant VMCUB2-1-1 (lane a), human tumor cell line VMCUB2 (b) and NIH 3T3 cells (c) were analyzed by formaldehyde-agarose gel electrophoresis transfer to nitrocellulose, and hybridization to the 32P-labelled VMCUB2-1 oncogene cDNA. The autoradiogram was exposed 3 hrs (lane a) and 24 hr (lanes b, c). Migration of 18S and 28S rRNAs are indicated.

Figure 5 shows structures and sequence of FGF-3 cDNA clones. A) The structure of the 1-2-2 cDNA clone corresponding to a transcript of a rearranged, activated human oncogene. Several restriction endonuclease cleavage sites are indicated: E = EcoRI, B = BamHI, S = SmaI, P = PstI. B) Structure of a homologous cDNA clone isolated from a library of human brain stem RNA (one day old, autopsy). The library was kindly provided by Dr. R. Lazzarini. The circled EcoRI sites at the left ends of both cDNAs are part of synthetic linker sequences added during cloning. C) Sequence of the 1-2-2 cDNA clone depicted in panel A. The nucleotide sequence (1120 bases) is shown along with the predicted amino acid sequences specified by the two open reading frames, ORF-1 and ORF-2. The homologous brain cDNA sequence is identical to bases 246-1120 depicted here.

Figure 6 shows the homology between the predicted FGF-3 protein and other FGF-related proteins. The predicted human FGF-3 amino acid sequence (from ORF-2) is aligned for maximum homology with sequences of the precursor proteins encoded by human hst/KS3, murine int-2, human basic FGF, and human acidic FGF. Amino acid positions of identity or conservative substitution between FGF-3 and related proteins are boxed and shaded. Conservative substitutions are defined as D = E, K = R, S = T, I = L = V, F = Y. Within regions 90-180 and 187-217 of the FGF-3 sequence, there is 50.4% sequence identity with hst/KS3, 47.5% identity with int-2, 43.4% with basic FGF, 40.2% with acidic FGF.

Figure 7 shows the structure of the rearranged, activated FGF-3 oncogene. A) Structural map of the rearranged FGF-3 gene. Thin hashmarks indicate positions of EcoRI cleavage sites, and the sizes of the EcoRI fragments (in kilobase pairs) are indicated above the map. The three exons are denoted by solid boxes marked I, II, and III. The precise locations of exons I and III were obtained by mapping restriction sites in the genomic DNA which correspond to sites in the cDNA clone. Exon II lies within the 5.8 kbp EcoRI fragment, but its precise positon was not determined (signified by the "?" symbol). The arrowhead marks the point of rearrangement between the native FGF-3 gene and plasmid pLTRneo. B) Sequences at the exon-intron boundaries. Exon sequnces are shown in capital letters, intron sequences in small case. Only sequences near the boundaries are shown. Exon seqeunces are numbered according to the corresponding FGF-3 1-2-2 cDNA clone sequence (Figure 1C). The corresponding amino acid sequence of FGF-3 is shown above exon sequences. C) Schematic diagram illustrating the rearrangement between the native FGF-3 gene and plasmid pLTRneo which generated the activated FGF-3 gene. Solid box indicates exon I of fGF-3 gene, rightward arrows marking site of FGF-3 transcription initiation. One of the two LTR sequences in pLTRneo is shown in open box, with its promoter initiating transcription from the leftward arrow. Diagonally shaded areas within LTR are the 75 base pair repeats which contain the enhancer. Vertical arrowheads mark breakpoints in pLTRneo and native FGF-3 gene accompanying rearrangement. E = EcoRI site, H = HindIII site. D) Sequence of rearranged FGF-3 gene in the promoter region. The sequences are numbered corresponding to the site of transcription initiation (+1), mapped by primer extension analysis. The arrow at +23 corresponds to the first base in the 1-2-2 cDNA clone (Figure 1C). A TATAA box presumptive promoter element (-31 to -27) is bracketed. LTR sequences in the rearranged gene lie upstream from -129. The direct repeat elements of the LTR are marked "DR".

Figure 8 shows mapping the 5' end of FGF-3 RNAs by primer extension analysis. Ten micrograms total cytoplasmic RNAs were annealed with the oligonucleotide 5' (32P) PO4-CCTAAGTGCATCTTGTA-3'OH complementary to FGF-3 RNA (bases 105-121 of 1-2-2 cDNA clone), and the mixture was incubated in buffer containing deoxynucleoside triphosphates and AMV reverse transcriptase. Reaction products were resolved on a 8 M urea - 12% polyacrylamide sequencing gel. RNAs from human tumor cell lines 639V (lane a) and SKHEP-1 (lane b) and from 3T3 transformant VMCUB2-1 (lane c) were used as templates. The same oligonucleotide (but unlabelled) was used to prime DNA sequencing reactions, using a subclone of the rearranged FGF-3 gene as template. Sequencing reactions are designated A, C, G, T corresponding to the sense strand of the FGF-3 gene, and lengths along the sequence ladder are numbered alongside the figure. A TATAA box upstream of the transcribed sequences is indicated.

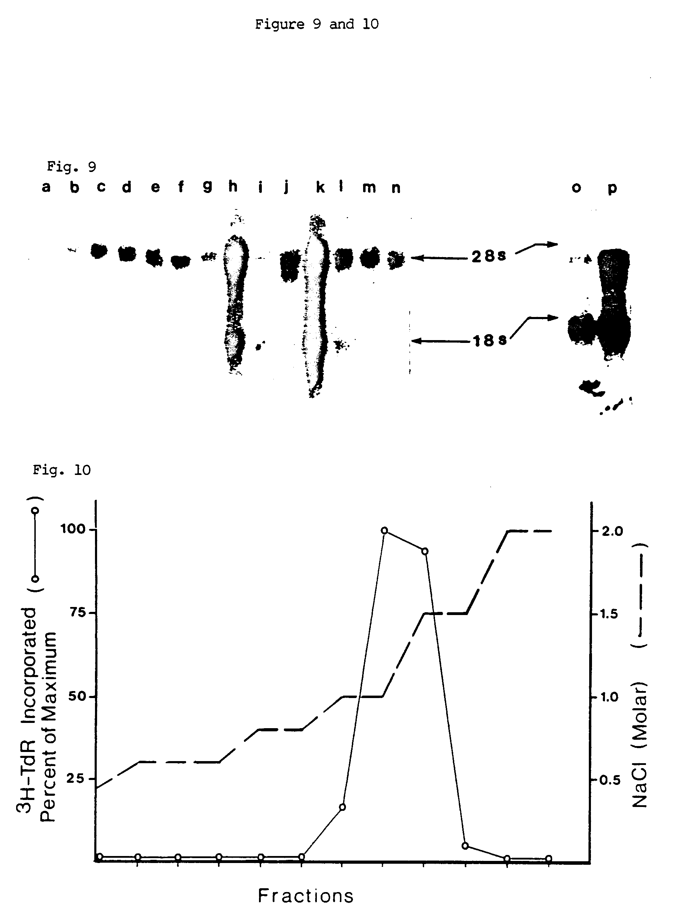

Figure 9 shows northern blot analysis of FGF-3 RNA in human tumor cell lines. Ten micrograms of total cytoplasmic RNAs (lanes a-n) or one microgram polyA-selected cytoplasmic RNAs (lanes o, p) were subjected to electrophoresis through 1.5% agarose gels containing 2.2 M formaldehyde. Gel-embedded RNA was transferred to nitrocellulose, hybridized with nick-translated FGF-3 1-2-2 cDNA, and autoradiographed. Total RNA from NIH 3T3 cells (lane a), human tumor cell lines VMCUB1 (b), VMCUB2 (c), Calu4 (d), KNS62 (e), BT20 (f), MDAMB469 (g), SKHEP-1 (h), MCF-7 (i), HEC-1A (j), 639V (k), 253J (1), HT29 (m), SH1 (n). PolyA RNA from 639V (o) and from NIH 3T3 transformant VMCUB2-1 bearing the rearranged, LTR-activated FGF-3 gene (p). The positions of 18S (1.9 kb) and 28S (4.5 kb) ribosomal RNAs are indicated.

Figure 10 shows Heparin Sepharose chromatography of mitogenic activity secreted from transformed cells expressing FGF-3. NIH 3T3 cells transformed by plasmid pLTR122 (containing FGF-3 cDNA linked to LTR sequences) were used to condition 300ml of serum-free medium. Conditioned medium was passed over a 1.0 ml Heparin-Sepharose column at room temperature, and the column was washed with 20 ml Tris (ph 7.5) buffer containing 0.45 M NaCl. Bound material was eluted stepwise with Tris-buffered NaCl solutions (0.6, 0.8, 1.0, and 2.0 M). Ten microliters of the 1 ml fractions were assayed for ability to stimulate DNA synthesis in quiescent Balb/c 3T3 cell cultures Tritiated thymidine incorporation data is expressed as percentage of maximum incorporation attainable in the assay using 10% calf serum (1.5x 106 cpm per 100,000 cells).

## Detailed Description of the Invention

This invention provides a purified polypeptide having the amino acid sequence shown in Figure 5 for either open reading frame (ORF) 2 or 1, and a purified nucleic acid molecule encoding the polypeptides. The purified nucleic acid molecule may be DNA or RNA.

This invention also provides a vector which comprises the nucleic acid molecule of this invention. This vector may be any vector known in the art including a plasmid or a virus. In a preferred embodiment, the plasmid is the plasmid designated pLTR-122 and deposited in an E. coli strain under ATCC No. 67413.

This invention also provides a host vector system for producing a polypeptide having the amino acid sequence shown in Fig. 5 for ORF-2. The vector comprises a plasmid in a suitable host. The suitable host may be a eucaryotic cell which in turn may be a mammalian cell. Preferably, the mammalian cell is a NIH 3T3 cell.

This invention further provides a method for producing a polypeptide having the amino acid sequence shown in Figure 5. The method comprises growing the host vector system of this invention so as to produce the polypeptide in the host and recovering the polypeptide so produced.

This invention still further provides a pharmaceutical composition comprising an effective amount of the polypeptide having the amino acid sequence shown in Figure 5 and a pharmaceutically acceptable carrier. A method of stimulating the proliferation of mesodermal cells is also provided. This method comprises contacting the mesodermal cells with an effective mesodermal cell proliferation-stimulating amount of the pharmaceutical composition. In a preferred embodiment the mesodermal cells are vascular endothelial cells. Additionally, the invention provides a method of stimulating capillary growth comprising contacting the capillaries with an effective capillary growth-stimulating amount of the pharmaceutical composition. Further, a pharmaceutical composition of this invention for promoting tissue repair in a subject with damaged tissue is provided.

This invention also provides an isolated fragment of total human genomic DNA comprising the oncogene encoding the amino acid sequence shown in Fig. 5 for ORF-2. Additionally, a nucleic acid molecule useful as a probe for detecting the oncogene is provided. This probe may be a polynucleotide or an oligonucleotide.

This invention still further provides a reagent comprising an antibody capable of specifically forming a complex with the polypeptide shown in Figure 5. The antibody may be a polyclonal antibody or a monoclonal antibody.

A reagent comprising an antibody capable of specifically forming a complex with the polypeptide shown in Fig. 5 for diagnosing in a subject a neoplastic condition associated with the presence of an activated oncogene is also provided. The subject may be a human being. Further, a method for detecting in a body fluid the precence of at least a portion of the polypeptide of this invention, wherein the detecting comprises contacting the polypeptide with a reagent of this invention to form an antibody-polypeptide complex, and detecting the complex so formed.

The invention discloses a reagent of this invention for use in the treatment of cancer.

The polypeptide encoded by ORF-1, or a functional equilivant thereof, may be used to influence the translation efficiency of the FGF-3 protein. This influence may either increase or inhibit the translation fo the FGF-3 protein.

### Materials and Methods

**Human Tumor Cell Lines.** Tumor cell lines were generously provided by Dr. Jorgen Fogh at the Sloan-Kettering Institute for Cancer Research, Rye, N.Y. Most of these cell lines have been described (19-24). Calu4 was derived from a small cell lung carcinoma by Dr. J. Fogh, and MDA-MB-453 was derived from a breast carcinoma by Dr. R. Cailleau.

Balb/c 3T3 cells, NIH 3T3 cells, and NIH 3T3 cells transformed by various oncogenes were previously described (18, 45). VMCUB2-1 are NIH 3T3 cells transformed with the rearranged, LTR-activated human FGF-3 gene; 3T3-LTR122 are NIH 3T3 cells bearing pLTR122, a plasmid containing 1-2-2 FGF-3 cDNA situated between two MLV LTR elements; 3T3-src and 3T3 ras are NIH 3T3 lines transformed by plasmids bearing the v-src and the mutant human c-H-ras (valine 12) oncogenes. Human tumor cell lines described in several references (19, 20, 21) were obtained from James Loveless at the Memorial Sloan Kettering Cancer Institute; VMCUB1, VMCUB2, 639V, and 253J are from bladder carcinomas, Calu4 and KNS62 from lung carcinomas, BT20, MCF-7 and MDAMB-469 from breast carcinomas, HT29 from a colon carcinoma, SH-1 from a melanoma, SKHEP-1 from a hepatoma, and HEC-1A from and endometrial carcinoma. Fetal bovine heart endothelial cells (FBHE) (46) were obtained from the American Type Tissue Culture Collection.

**Preparation of Defined Medium**. Defined medium was prepared as described previously (18) by adding supplements to a 3:1 mixture of Dulbecco's medium (DME) and Ham's F12 nutrient mixture, and medium was not used beyond one month after preparation. These supplements were 8 mM NaHC03, 15 mM HEPES pH 7.4, 3 mM histidine, 4 microMolar MnC12, 10 microMolar ethanolamine, 0.1 microMolar selenous acid (sodium salt), 2 microMolar hydrocortisone, 5 micrograms/ml transferrin, 500 micrograms/ml bovine serum albumin/linoleic acid complex, and 20 micrograms/ml insulin. Medium was prepared in autoclaved, water-rinsed glass bottles that had not been exposed to serum nor detergents, and was transferred with sterile plastic pipets. Medium exposed to certain plastic vessels became toxic to cells, and, when necessary, defined medium was stored exclusively in Corning polystyrene tubes or flasks.

**DNA Preparation Methods.** Mammalian cell DNAs and bacterial plasmid and bacteriophage DNAs were prepared by standard methods (2,25,26).

**DNA Transfection and Defined Medium Selection.** DNA transfection and selection for transformed clones followed the scheme illustrated in Figure 1. NIH 3T3 cells were transfected under standard conditions (27) with 30 micrograms cellular DNA and one microgram of plasmid pLTRneo (18) per 100 mm dish, then passaged 1:2 and cultured in DME with serum plus 1 mg/ml G418 for approximately 12 days to select colonies which had acquired pLTRneo and, by cotransfer (27), the transfected cellular DNA. Colonies on each pair of culture dishes were trypsinized and pooled, and 100,000 cells in DME + serum were plated onto poly-D-lysine/fibronectin coated dishes (Nunc) as previously described (18). After cell attachment, cultures were refed with the defined medium described above. Cultures were refed with the same medium the next day and thereafter on a three day schedule until all normal cells died and transformed colonies, if any, had developed (7-10 days). Transformed colonies were individually trypsinized and expanded in DME plus serum. Transformed cultures were freed of any residual normal cells by replating on fibronectin-coated dishes and maintaining in defined medium.

**Plasmids Employed for Nucleic Acid Hybridization.** Plasmids were provided by Drs. M. Wigler, P. Besmer, F. Alt, R. Axel, and R. Parker. Plasmid clones employed were human H-ras, N-ras, N-myc, rho, murine c-myc, and viral K-ras, mos, src, fos, sis, ski, myb, rel, fps/fes, raf/mil, fms, erbAB, fgr. Blur-2 contains a human Alu repeat sequence (30), which we cloned into the BamHI site of pSP64 (31) to generate pSP6-Alu.

**Filter-blotted DNA Hybridization.** DNAs were digested with restriction endonucleases, subjected to agarose gel electrophoresis, and transferred to nitrocellulose filters by the method of Southern (32). DNA probes were radiolabelled with 32P by nick translation (33), and 32P-labelled RNA was transcribed from EcoRI-linearized pSP6-Alu DNA using SP6 polymerase (31). Hybridizations with Sp6-derived Alu repeat sequence probes were conducted at 43 degrees C in the presence of 50% formamide and 10% dextran sulfate (34), while hybridizations with DNA probes were conducted at 70 degrees C without formamide/dextran sulfate (2).

**Filter-blotted RNA Hybridization.** Preparations of cytoplasmic RNA (35) were subjected to electrophoresis through formaldehyde agarose gels, transferred to nitrocellulose, and hybridized by standard procedure (34).

**Construction and Screening of Genomic DNA and cDNA Libraries in Bacteriophage Lambda.** 15 to 20 kilobase pair DNA fragments purified from EcoRI restriction endonuclease digestion of cellular DNAs were ligated to Charon 4A or EMBL3 bacteriophage vectors, packaged into virions, and screened by standard procedure (36, 37). cDNAs were synthesized from polyA + RNA, using RNase and DNA polymerase to synthesize second strands (38). cDNAs were ligated to EcoRI linkers and cloned into bacteriophage Charon 16A.

**Tumorigenicity of Transformed Cell Lines.** Tumorigencity of transformed cell lines in immunodeficient nude mice followed procedures described elsewhere (39) and outlined in the legend to Table 3.

**Construction of Expression Vector.** pvcos-7 (obtained from Stephen Goff and Leslie Lobel, Columbia University) is a cosmid with ampicillin resistance, containing a modified Moloney murine leukemia provirus. The provirus has been modified by a deletion of the nucleotide sequence from the Pst1 site (at map position 1.0 kbp) to the Hpa 1 site (at map position 7.6 kbp). An EcoRI linker has been inserted at the cite of the deletion. The pVCOS-7 and the FGF-3 cDNA were both digested with EcoRI. The FGF-3 cDNA was then cloned into the pvcos-7 by standard procedures. The resulting vector was designated pLTR-122. pLTR-122 was then transfected into NIH 3T3 cells by the method described by Wigler et al. (44). The colonies were then trypsinized, pooled and assayed for oncogene transformed cells by a defined medium culture assay (18).

**FGF-3 Genomic and cDNA Clones.** NIH 3T3 cells transformed by the rearranged FGF-3 gene (the VMCUB2-1 cell line) had been used to clone the FGF-3 gene in lambda vector EMBL4, and these cells were also used to derive the biologically active cDNA clone 1-2-2. An FGF-3 cDNA clone was also obtained by screening a lambda gtll cDNA library derived from RNA of human brain stem (1-day old autopsy) (library kindly provided by Dr. R. Lazzarini). Both cDNAs were subcloned into plasmid pUC8 by EcoRI digestion, which cleaved the cDNAs at a native EcoRI cleavage site 3' to the coding sequences; hence the cDNAs lack their 3' ends and polyA tails. For the purpose of DNA sequencing, fragments of cDNA and genomic clones were subcloned into pUC8 following restriction enzyme digestion or mild DNase I treatment.

**DNA Sequencing.** Plasmid inserts were sequenced by modification of the standard DNA polymerase dideoxynucleotide chain termination method (47). One to two micrograms plasmid was denatured with alkali, neutralized in the presence of 25 nanograms oligonucleotide primer, and ethanol precipitated. Pellets were dissolved and sequenced with T7 DNA polymerase (Sequenase®, U.S. Biochemical), using alpha-35S-dATP as the labelled nucleotide. The primers most often used were 17-mers complementary to pUC8 sequences flanking the inserts. The oligonucleotides 5'CCTAAGTGCATCTTGTA-3' and 5'ACTTGCATGGAGTTTTC3', complementary to bases 105-121 and 579-595 of the 1-2-2 FGF-3 cDNA clone, were used in certain experiments. The cDNAs were fully sequenced on both strands, as was the 5' region of the FGF-3 gene. For the purpose of mapping the exon-intron boundaries, certain genomic clones were sequenced on one strand only.

**Analysis of FGF-3 RNA.** Cytoplasmic RNAs were extracted from cultured cells (35) and in some casess enriched for polyadenylated RNA by oligo-dT cellulose chromatography. RNAs were assayed for FGF-3 transcripts by formaldehyde agarose gel electrophoresis and filter blot hybridization, using standard procedure (34). Ethidium bromide (50 ng/ml) was included in gels to allow visualization of ribosomal RNAs before blotting.

Determination of FGF-3 RNA 5, ends were made by standard primer extension procedure (48). The oligonucleotide 5' CCTAAGTGCAGCTTGTA 3' complementary to bases 105-121 of the 1-2-2 cDNA clone was 5'-end-labelled with gamma-32P-ATP and polynucleotide kinase, and 15 nanograms primer was annealed with ten micrograms polyadenylated cytoplasmic RNAs. The mixture was precipitated, redissolved, incubated with dNTPs and AMV reverse transcriptase (Boehringer Mannheim®), and analyzed by electrophoresis through a 8M urea - 12% polyacrylamide gel followed by autoradiography. DNA sequencing reactions using the same oligonucleotide primer and FGF-3 genomic DNA template were run alongside to provide size markers.

**Mitogenic Assays.** Transformed NIH 3T3 cells were maintained at high density in a defined medium containing insulin, but lacking platelet-derived growth factor or FGFs (18). After two to three days conditioning, media was harvested for testing of mitogenic activity towards Balb/c 3T3 fibroblasts. To test for activity towards endothelial cells, transformed cells were maintained in DME supplemented with 10 micrograms per ml heparin.

For the fibroblast mitogenic assay, culture wells were seeded to 10% confluence with Balb/c 3T3 murine fibroblasts, and were rendered quiescent by maintaining without refeeding for five days, as originally described (49). Cultures were refed with dilutions of conditioned medium (diluted with defined medium containing insulin), 4 $\mu$Ci/ml 3H-thymidine was added 15 hours later, and thymidine incorporation into DNA was measured 3 hours later by fixing cultures in 15% trichloroacetic acid, washing with water, dissolving in 0.5 M NaOH, and counting by liquid scintillation.

For the endothelial cell mitogenic assay, FBHE endothelial cells were plated at 40,000 cells per 60 mm dish in DME + 3% calf serum. The next day, an equal volume of DME + 10 $\mu$g/ml heparin contianing various dilutions of conditioned medium was added, and cell number was determined six days later by trypsinization and counting by hemocytometer.

**Heparin Affinity Chromatography.** NIH 3T3 cells transformed with the plasmid pLTR122 were used to condition DME medium containing no supplements. Three hundred milliliters of 48 hr. conditioned medium was passed directly through a 1 ml. column of heparin-Sepharose (Pharmacia®) at room temperature. The column was washed extensively with 0.45 M NaCl - 20 mM Tris pH 7.5, then washed in succession with tris buffer containing NaCl at 0.6 M (3 ml), 0.8 M (2 ml), 1.0 M (2 ml), 1.5 M (2 ml), and 2.0 M (2 ml). One ml fractions were collected, and fractiions were diluted 1:100 with defined medium containing insulin (18) and assayed for stimulation of Balb/c 3T3 cells.

Results

**Defined Medium Transformation Assay.** NIH 3T3 cells can grow efficiently in a basal medium supplemented with transferrin, insulin, and fibroblast growth factor (FGF) or platelet-derived growth factor (PDGF), but die in the absence of FGF and PDGF. By contrast, ras-, sis-, src-, and mos-transformed 3T3 cells proliferate in the PDGF/FGF-free defined medium (18). We have developed a transformation assay based upon cell growth in PDGF/FGF-free defined medium (see Figure 1 and Methods). Culture of 3T3 cells are transfected with cellular DNA and pLTRneo, selected with neomycin analog G418 to enrich for cells with stabily acquired foreign DNA, and then selected in PDGF/FGF-free defined medium.

We have performed transfections with DNAs from seventeen human tumor-derived cell lines, and have used human placental and NIH 3T3 DNAs as negative controls. The results are tabulated in Table 1.

Table 1

| Selection of Transformed Cells Following Transfection if NIH 3T3 Cells with NDAs from Human Tumor Cell Lines | | | | | |
|---|---|---|---|---|---|
| Tumor Cell Line | Tissue of Origin | #G418r Colonies Screened | Number of Independent Transformants | Transformants per 10,000 G418r Colonies | Ras Homology |
| Calu-4 | Lung | 8,000 | 8 | 10 | K-ras |
| KNS 62 | Lung | 9,500 | 4 | 4.1 | H-ras |
| SAOS-2 | Bone | 15,000 | 4 | 2.7 | None |
| 639 V | Bladder | 15,000 | 2 | 1.3 | K-ras |
| Sk-N-MC | Neural | 29,000 | 4 | 1.3 | None |
| VM-CUB-2 | Bladder | 19,000 | 2 | 1.1 | None |
| SK-MES-1 | Lung | 53,000 | 4 | 0.8 | None |
| SW 1088 | Astrocyte | 22,000 | 1 | 0.5 | None |
| A 172 | Neural | 9,000 | 0 | | |
| BT-20 | Breast | 9,000 | 0 | | |
| HT 1376 | Bladder | 8,000 | 0 | | |
| 5637 | Bladder | 6,500 | 0 | | |
| MCF-7 | Breast | 8,000 | 0 | | |
| SK-HEP-1 | Liver | 7,000 | 0 | | |
| VM-CUB-1 | Bladder | 6,500 | 0 | | |
| IMR-32 | Neural | 9,000 | 0 | | |
| MDA-MB-453 | Breast | 8,000 | 0 | | |
| Nontransformed Cells | | | | | |
| NIH 3T3 | | 95,000 | 0 | 0.1 | |
| Human placenta | | 84,000 | 1 | 0.1 | |

Table 1. NIH 3T3 cells transfected with tumor cell DNA and pLRneo were first selected for G418 resistance in serum-containing medium. The number of G418r colonies were approximated prior to pooling and culturing in PDGF/FGF-free defined medium. Transformed colonies were considered to derive from independent transfection events if they arose on separate defined media dishes. Transformants containing human ras oncogenes are indicated.

DNAs from eight of the tumor cell lines gave transformants upon transfection, with frequencies ranging from greater than ten transformants per 10,000 G418-resistant colonies screened for Calu-4 lung carcinoma DNA to 0.5/10,000 for SW1088 astrocytoma DNA. Transfections with normal human and mouse DNAs have yielded one transformant for 179,000 G418-resistant colonies screened.

DNA was prepared from transformants derived by transfection with human tumor cell line DNAs. These transformant DNAs were used in a second cycle of 3T3 cell transfection, G418 selection, and defined medium selection. As shown in Table 2, most transformant DNAs generated secondary tranformants upon transfection.

Table 2

| Selection of Transformed Cells Following Transfection of NIH 3T3 Cells with DNAs from Primary Transformant Cell Lines | | | |
|---|---|---|---|
| Primary Transformant Cell Line | # G418r Colonies Screened | Number of Secondary Transformants | Transformants per 10,000 G418r Colonies |
| SAOS2-2 | 16,000 | 0 | 1 |
| SAOS2-3 | 5,000 | 2 | 4.0 |
| VMCUB2-1 | 6,500 | 3 | 4.7 |
| VMCUB2-2 | 13,000 | 4 | 3.1 |
| SKNMC-1 | 3,000 | 3 | 10.0 |
| SKNMC-2 | 6,500 | 3 | 4.7 |
| SKNMC-3 | 9,500 | 0 | 1 |
| SKMES-1 | 6,000 | 1 | 1.7 |
| SMKES-2 | 7,000 | 4 | 5.7 |
| SKMES-3 | 8,500 | 3 | 3.6 |
| Table 2. Primary transformant cell lines derived from transfection and defined medium selection as listed in Table 1. E.g., VMCUB2-2 derived from transfection with DNA from human tumor cell line VMCUB2. Transfection and selection procedures were as described in Table 1. | | | |

In some instances, DNAs from secondary transformants were transfected into 3T3 cells to generate tertiary transformants (data not shown).

**Tumorigenicity of Transformed Cell Lines.** Seven transformed cell lines which derived from transfection with DNAs from four human tumor cell lines were tested for tumorigenicity. Trypsinized cell suspensions were inoculated into athymic nu/nu (nude) mice, which were monitored for tumor development. As shown in Table 3, six of the seven transformed cell lines were strongly tumorigenic, inducing progressive tumors in all inoculated mice within two weeks.

Table 3

| Tumorigenicity of Transformed Cell Lines in Nude Mice | | |
|---|---|---|
| Transformant Inoculated | No. Mice with Tumors/No. Mice Inoculated | Latency (weeks) |
| SAOS2-3-1 | 3 / 3 | 2 |
| SAOS2-3-1-1 | 3 / 3 | 1-2 |
| VMCUB2-1-1 | 3 / 3 | 1-2 |
| VMCUB2-1-2 | 1 / 3 | 6 |
| SKMES-4 | 3 / 3 | 1-2 |
| SKMES-5 | 3 / 3 | 1-2 |
| SKNMC-3 | 3 / 3 | 1-2 |
| NIH 3T3 | 0 / 3 | No tumors after 11 weeks |

Table 3. Transformed cell lines and normal NIH 3T3 cells were trypsinized, counted, and inoculated subcutaneously into athymic nu/nu mice, as previously described (54). Two million cells were injected per mouse, except for cell lines VMCUB2-1-2 and SKMES-4, for which one million cells were used. Mice were monitored weekly for appearance and progression of tumors at site of inoculation. The latency period is the average period for appearance of progressive tumors.

The seventh transformant was weakly tumorigenic, while normal NIH 3T3 cells were not tumorigenic over the eleven week monitoring period. Hence, the transfected genes which confer growth factor independence also confer oncogenic potential to 3T3 cells.

**Structure of the Oncogene in a Transformant Derived by Transfection with VMCUB2 Bladder Carcinoma DNA.** Four secondary transformants derived from primary transformant VMCUB2-2 lack Alu repeats (Figure 2, lanes b-e) while three transformants derived from primary VMCUB2-1 each have two Alu sequences (lanes g-i), one of which is within a conserved 2.3 kbp EcoRI DNA fragment. Hence, different molecular events gave rise to the two primary VMCUB2 transformants. The human oncogene in transformant VMCUB2-1 has been cloned from a phage genomic library of secondary transformant VMCUB2-1-1. Initial clones were obtained by Alu sequence homology, and further clones were obtained by genomic walking.

A physical map of the FGF-3 oncogene is shown in Figure 3. The genomic inserts in the RA3 and R4 phage DNA isolated lack transforming activity. However, a ligated mixture of RA3 and R4 partial EcoRI digests gave many transformed colonies following transfection (data not shown). This demonstrates that these two overlapping clones span the entire oncogene. As shown in Figure 3, three genomic EcoRI fragments in the gene hybridize to a 1.1 kbp cDNA corresponding to the transcript of this gene. This cDNA has transforming activity when fused to a retroviral promoter. We have hybridized this cDNA clone at low stringency to a panel of eighteen known oncogenes and have failed to detect sequence homology.

The coding strand of the cDNA was determined by sequencing the long open reading frame (see below), and the 5'-3' orientation of the genomic DNA map was determined by mapping genomic restriction enzyme sites corresponding to sites on the cDNA.

Surprisingly, we found that the 2.8 kbp EcoRI fragment at the 5' end of the oncogene locus hybridized to the identically sized RI fragment of pLTRneo (Figure 3). Further mapping of restriction sites at the 5' end of the oncogene allowed us to conclude that transfection of VMCUB2 bladder carcinoma DNA together with pLTRneo had resulted in a fortuitous DNA rearrangement, whereby the LTRneo plasmid became linked to the 5' end of the oncogene in a 5'-5' orientation. The breakpoint between human and plasmid DNAs is located within the 1 kbp region between the XbaI site in LTRneo and the PstI site in the 5' transcribed region of the human gene.

This rearrangement probably results in increased oncogene expression due to the influence of the retrovirus enhancer element in pLTRneo. This is supported by RNA filter blot analysis, using the oncogene cDNA as hybridization probe. As shown in Figure 4, the cDNA detects two abundant transcripts (@18S and @28S) in a transformant bearing the FGF-3 oncogene (lane a). This expression is dramatically higher (approximately 50-fold) than that seen in the human bladder carcinoma cell line VMCUB2 (lane b). The sizes of transcripts in the human and transformed cells are approximately the same, suggesting that LTR-enhanced transcription proceeds from the native human promoter(s).

**RNA Transcribed from the Rearranged Oncogene Encodes a Protein Related to Fibroblast Growth Factors.** One oncogene cDNA (clone 1-2-2) of 1121 base pairs has been completely sequenced and is presented in Figure 5. The 1120 base pair sequence lacks the 3' polyA tract, because the cDNA cloning procedure involved EcoRI digestion which cut the native cDNA at least once. The single strand shown corresponds to that of the RNA, as demonstrated by the ability of an oligonucleotide of complementary sequence to prime reverse transcription of the oncogene-encoded RNA (see below). The cDNA sequence contains two ATG-initiated open reading frames (ORFs); ORF-1 and ORF-2 can specify polypeptides of 38 and 267 amino acid residues, respectively. The two reading frames slightly overlap, with the ORF-1 termination codon, TGA, situated one nucleotide downstream of the ORF-2 initiator ATG.

The protein specified by ORF-2 bears a leucine-rich hydrophobic amino terminus, which may serve as a signal sequence for cotranslational transport into the endoplasmic reticulum (53). The protein's lack of other extensive tracts of hydrophobic residues suggests that the ORF-2 product can be secreted. The predicted protein also bears a consensus sequence for N-linked glycosylation, AsnGlySer (109-111). When the ORF-2 protein sequence was compared with sequences in the PIR-NBRF (Protein Identification Resource, National Biomedical Research Foundation) database, substantial homology was detected between the ORF-2 protein and both acidic and basic fibroblast growth factors. The recently described int-2 and hst/KS3 predicted protein sequences are also homologous to, but distinct from, the ORF-2 protein, which we here term FGF-3.

A comparison of the FGF-3 amino acid sequence with those for other FGF family proteins is shown in Figure 6. Two blocks of FGF-3 amino acid residues (90-180, 187-207) show substantial homology to the other proteins, ranging from 40.2% (versus acidic FGF) to 50.4% (versus hst/KS3). Within these homology blocks, the five proteins are identical at 20% of the residues, and allowing for conservative amino acid substitutions, the five proteins share 29% homology. Nucleotide sequence homology between the FGF-3 coding sequences and those of the related genes is minimal.

The sequences of the FGF-related proteins differ in several respects. First, the five sequences differ in the length and sequence of residues between the two homology blocks and distil to them. Second, the FGF-3 sequence is unique in bearing an insertion within the second homology block (CysSer, 201-202). Lastly, the amino-terminal sequences of the FGF-3, hst/KS3, and int-2 proteins are extensively hydrophobic, while those of acidic and basic FGF precursor proteins are not, suggesting differences in post-translational trafficking amongst the FGF-like proteins.

**The Structure of the FGF-3 Oncogene is Similar to Those of Other FGF-Related Genes.** The structure of the rearranged, transforming FGF-3 oncogene is illustrated in Figure 7, Panel A. The portion of the oncogene's transcripts represented in the transforming cDNA clone 1-2-2 derive from three exons in the gene. The DNA sequences at the exon-intron boundaries and their positions with respect to the coding sequence are presented in panel B of Figure 7. Exon I spans coding sequences from the initiation codon through the first nucleotide of serine codon 118, while exon II terminates after serine codon 152. Since the splice boundaries are at points within the FGF family homology blocks, we could compare these exon boundaries to those within the hst/KS3, int-2, and basic FGF genes (54, 55, 56). The exon boundaries of both the hst/KS3 and int-2 oncogenes are positioned identically to those of the FGF-3 gene. The exon II/III boundary of the basic FGF gene also aligns perfectly, while the exon I/II boundary of this gene is shifted by three nucleotides. Hence, the FGF-related genes have evolved with virtually no deviation from their ancestral gene's exon structure. The FGF-related genes bear introns of dramatically different lengths: hst/KS3 introns total 1.1 kbp, FGF-3 introns total 19 kbp, and basic FGF gene introns exceed 30 kbp.

**Expression of the Native FGF-3 Gene.** We have examined whether the native FGF-3 gene specifies the same RNA transcripts and protein as does the rearranged, LTR-activated gene. One approach we have used has been to characterize cDNA clones corresponding to transcripts of the native FGF-3 gene. Based upon preliminary data suggesting expression of FGF-3 in fetal brain, we chose to screen a cDNA library constructed in lambda gtll vector using RNA from autopsy of one-day old human brain stem (library kindly provided by Dr. R. Lazzarini). Four clones hybridizing to the FGF-3 gene were detected upon library screening, one of which was subcloned and fully sequenced. This brain cDNA clone was 245 nucleotides shorter than the 1-2-2 FGF-3 cDNA clone (Figure 5B), but the sequence of the brain cDNA was identical to that of bases 246 to 1120 in the 1-2-2 clone's sequence (Figure 5C).

We have also loked for expression of FGF-3 within a panel of human tumor cell lines of solid tumor origin. Cytoplasmic RNAs were prepared from thirteen such cell lines, and the samples were assayed for FGF-3 transcripts by gel electrophoresis and filter blot hybridizaiton. None of htese cell lines had shown any evidence for FGF-3 gene rearrangement (data not shown). Figure 9 shows that two of the cell lines, hepatoma SKHEP1 and bladder carcinoma 639V, express two RNA species homologous to the FGF-3 cDNA probe (lanes h,k). A third cell line, endometrial carcinoma HEC-1A, expressed FGF-3 RNA at lower levels (lane j), while the other tumor cell lines did not express FGF-3 detectably. (The cDNA probe also hybridized weakly to human 28S ribosomal RNA.)

The FGF-3 transcripts in the tumor cell lines were indistiguishable in size and relative abundance from the two RNA species transcribed from the rearranged, LTR-activated FGF-3 gene in transformed 3T3 cells (Figure 9, lanes o, p). By primer extension analysis, we could show that at least one of the native FGF-3 transcripts in SKHEP1 and 639V cells has precisely the same 5' end as that which was characterized for a transcript of the rearranged gene (Figure 8, lanes a, b). Taken together, these data confirm that RNA species encoded by native and rearranged FGF-3 genes are the same.

**FGF-3 Transformed 3T3 Cells Secrete a Mitogen Functionally Related to FGFs.** Our sequence data suggests that the FGF-3 gene encodes a secreted growth factor. We have linked the FGF-3 cDNA clone to the mammalian expression plasmid vector pvcos-7. This construct, termed pLTR-122, transforms fibroblasts with high efficiency. pLTR-122 has been transfected into NIH 3T3 cells to derive transformants which express the oncogene and secrete the FGF-3 encoded growth factor.

We have assayed for mitogenic activity secreted from 3T3 cells transformed by the rearranged FGF-3 gene or by FGF-3 cDNA linked to MLV-LTR sequences. Conditioned media from such transformed cell cultures (termed VMCUB2-1 and 3T3-LTR122, respectively) were serially diluted and assayed for the ability to stimulate DNA synthesis in quiescent Balb/c 3T3 fibroblast cultures. Table 4 shows that these transformed cells secrete mitogenic activity detectable at 1:8 dilutions. Secretion of mitogenic activity is not a property of transformed cells per se, as NIH 3T3 cells transformed by activated human H-ras or viral src oncogenes released little or no mitogenic activity (Table 4).

Table 4

| Stimulation of Quiescent Balb/c 3T3 Cells with Conditioned Media | | |
|---|---|---|
| Conditioned Medium From | Dilution | 3H-Thymidine Incorporation (cpm/10,000 |
| 3T3-ras | 1:2 | 1.1 |
| | 1:4 | 1.5 |
| | 1:8 | 1.0 |
| 3T3-src | 1:2 | 1.5 |
| | 1:4 | 1.1 |
| | 1:8 | 1.1 |
| VMCUB2-1 | 1:2 | 23.0 |
| | 1:4 | 9.5 |
| | 1:8 | 3.2 |
| 3T3-LTR122 | 1:2 | 130.0 |
| | 1:4 | 99.1 |
| | 1:8 | 40.8 |
| No conditioned medium | | 0.9 |
| With 10% calf serum | | 146.0 |

Balb/c 3T3 cells were plated in culture wells (20,000 cells/ 2.0 sq. cm. well) in serum-containing medium and maintained five days without refeeding, allowing the cells to form quiescent, serum-exhausted monolayers. Cultures were refed with serum-free medium containing dilutions of conditioned medium from transformed cells. 3H-thymidine (4 µCi/ml) was added 15 hrs. later, and after three hour incubation, cultures were fixed in 15% trichloroacetic acid, their DNA dissolved in 0.5 N NaOH, and incorporated label was assayed by liquid scintillation.

As a means of assessing whether the mitogen secreted by FGF-3 transformed cells is, indeed, FGF-3, we have tested whether this mitogen has properties diagnostic for FGFs. One property of acidic and basic FGFs is their ability to strongly bind to the glycosaminoglycan heparin (50, 51, 52). Elution of FGFs from heparin affinity resins requires NaCl concentrations of 1.0 M or greater. By contrast, platelet-derived growth factor, a basic protein which binds heparin by weaker ionic interactions, elutes at approximately 0.5 M NaCl. Mitogenic conditioned medium from FGF-3 cDNA-transformed 3T3 cells was passed directly over a heparin-Sepharose column, which was washed extensively with buffered 0.45 M NaCl, and then eluted with stepwise increasing salt concentrations. Dilutions of column fractions were assayed for the ability to stimulate quiescent Balb/c 3T3 cells. As shown in Figure 10, the peak of mitogenic activity eluted in the 1.0 and 1.5 M NaCl fractions.

A second property of FGFs is their broad spectrum of mitogenicity, including their activity towards vascular endothelial cells. Conditioned medium from FGF-3 transformed cells was tested for ability to stimulate proliferation of bovine heart endothelial cells. Table 5 shows that the conditioned medium stimulated endothelial cell growth as effectively as could partially purified basic FGF. These data strongly suggest functional similarity between FGF-3 and the well characterized fibroblast growth factors.

Table 5

| Factor Released from FGF-3 Transformed Cells Stimulates Endothelial Cell Growth | |
|---|---|
| Culture Medium Supplement | Number of Cell Doublings |
| None | 1.5 |
| 50 ng/ml FGF | 3.1 |
| Conditioned medium from 3T3-LTR122 cells | |
| 1:2 dilution | 3.3 |
| 1:8 dilution | 3.4 |

Fetal bovine heart endothelial cells (FBHE) were plated onto 60 mm tissue culture dishes (40,000 cells per dish) using DME medium containing 3% calf serum. After 24 hours, two plates of cells were individually trypsinized, and cell numbers determined by hemocytometer (60,000 cells +/- 10%). The media on other plates was diluted with an equal volume of DME + 10 micrograms/ml heparin + test substance. Cultures were maintained without refeeding for six days, and cell counts were determined by hemocytometer after trypsinization. Test substances were either crude FGF (Collaborative Research) (50 ng/ml final concentration) or dilutions of medium conditioned by the 3T3-LTR122 FGF-3 transformed cell line. Growth is expressed as number of cell doublings over six day period.

**Expression of FGF-3 Protein in Bacteria.** The 1-2-2 cDNA clone (the complete 1120 base pair EcoRI fragment) of the FGF-3 mRNA was cloned into the EcoRI restriction site of the bacterial expression vector pATH3 (57). The resultant construct, termed pTrpE-FGF-3, will direct synthesis in E. coli of a fusion protein containing the bacterial TrpE protein linked to the FGF-3 amino acid sequence. pTrpE-FGF-3 was introduced into E. coli HB101, and HB101 carrying pATH3 served as negative control. Five hundred milliliter cultures were harvested and sonicated in five milliliters buffer. Sonicates were clarified by centrifugation, and the soluble extracts were tested for ability to stimulate DNA synthesis in quiescent Balb/c 3T3 cells (protocol described in original manuscript). As shown in Table 6, three microliter of extract from HB101 bearing pTrpE-FGF-3 gives maximal stimulation of DNA synthesis, while comparable volume of extract from HB101 bearing pATH3 has no stimulatory effect. Furthermore, the mitogenic activity in HB101/pTrpE-FGF-3 extracts binds to heparin Sepharose columns and elutes at the same high salt concentrations as the growth factor secreted from NIH 3T3 cells transformed with the FGF-3 gene (pLTR122) (data not shown). Hence, the FGF-3 protein is conclusively shown to be a growth factor active on Balb/c 3T3 fibroblasts.

Table 6

| Mitogenic activity of bacterial extracts containing FGF-3 | | |
|---|---|---|
| Bacterial Extract | Volume of Extract (microliters) | DNA Synthesis (3H-TdR Incorporation) (c.p.m./1000) |
| HB101/pTrpE-FGF-3 | 1 | 105 |
| HB101/pTrpE-FGF-3 | 3 | 140 |
| HB101/pATH3 | 3 | 1.2 |
| NONE | | 1.1 |

References

1. Shih, C., Shilo, B.Z., Goldfarb, M.P. Dannenberg, A., Weinberg, R.A. (1979) Proc. Natl. Acad, Sci. USA 76, 5714-5718.
2. Perucho, M., Goldfarb, M.P., Shimizu, K., Lama, C., Fogh, J., Wigler, M.H. (1981). Cell 27, 467-476.
3. Murray, M., Shilo, B., Shih, C., Cowing, D., Hsu, H.W., Weinberg, R.A. (1981) Cell 25, 355-361.
4. Krontiris, T.G., Cooper, G.M. (1981) Proc. Nat. Acad. Sci. USA 78, 1181-1184.
5. Pulciani, S., Santos, E., Lauver, A.V., Long, L.K., Aaronson, S.A., Barbacid, M. (1983) Nature 300, 539-542.
6. Yuasa, Y., Srivastava, S., Dunn, D.Y., Rhim, J.S., Reddy, E.P., Aaronson, S.A. (1983) Nature 303, 775-779.
7. Parada, L.F., Tabin, C.J., Shih, C., Weinberg, R.A. (1982). Nature 297, 474-475.
8. Santos, E., Tronick, S.R., Aaronson, S.A. Pulciani, S., Barbacid, M. (1982) Nature 298, 343-347.
9. Der, C.J., Krontiris, T.G., Cooper, G.M. (1982) Proc. Nat. Acad. Sci. USA 79, 3637-3640.
10. Shimizu, K., Goldfarb, M., Suard, Y., Perucho, M., Li, Y., Kamata, T., Feramisco, J., Stavnezer, E., Fogh, J., Wigler, M. (1983) Proc. Nat. Acad. Sci. USA 80, 2112-2116.
11. Hall, A., Marshall, C.J. Spurr, N.K., Weiss, R.A. (1983) Nature 303, 396-410.
12. Shimizu, K., Nakatsu, Y., Sekiguchi, M. Hokamura, K., Tanaka, K., Terada, M., Sugimura, T. (1985) Proc. Nat. Acad. Sci. USA 82, 5641-5645.
13. Fukui, M., Yamamoto, T., Kawai, S., Maruo, K., Toyoshima, K. (1985) Proc. Nat. Acad. Sci. USA 82, 5954-5958.
14. Eva, A., Aaronson, S.A. (1985) Nature 316, 273-275.
15. Martin-Zanca, D., Hughes, S.H., Barbacid, M. (1986) Nature 319, 743-748.
16. Dean, M., Park, M., LeBeau, M.M., Robins, T.S., Diaz, M.O., Rowley, J.D. Blair, D.G., VandeWoude, G.F. (1985) Nature 318, 385-388.
17. Young, D., Waitches, G., Birchmeier, C., Fasano, O., Wigler, M. (1986) Cell 45, 711-719.
18. Zhan, Z., Goldfarb, M. (1986) Mol. Cell Biol. 6, 3541-3544.
19. Fogh, J. (1978) Nat. Cancer Inst. Monogr. 49, 5-9.
20. Fogh, J., Wright, W.C., Loveless, J.D. (1977) J. Nat. Cancer Inst. 58, 209-214.
21. Fough, J., Fogh, J.M., Orfeo, T. (1977) J. Nat. Cancer Inst. 59, 221-225.

22. Soule, H.D., Vasquez, J., Long, A., Albert, S., Brennan, M. (1973) J. Nat. Cancer Inst. 51, 1409-1416.
23. Takaki, T. (1980) J. Cancer Res. Clin. Oncol. 96, 27-33.
24. Rasheed, S., Gardner, M.B., Rongey, R.W., Nelson-Rees, W.A., Arnstein, P. (1977) J. Nat. Cancer Inst. 58, 881-890.
25. Tanaka, T., Weisblum, B., (1975) J. Bacteriol. 121, 354-362.
26. Yamamoto, K.R., Alberts, B.M., Benzinger, R., Lawhorne, L., Treiber, G. (1970) Virology 40, 734-744.
27. Wigler, M., Sweet, R., Sim, G.K., Wold, B., Pellicer, A., Lacy, E., Maniatis, T., Silverstein, S., Axel, R. (1979) Cell 16, 777-785.
28. Fasano, O., Taparowsky, E., Fiddes, J., Wigler, M., Goldfarb, M. (1983) J. Mol. App. Genet. 2, 173-180.
29. Ellis, R.W., Defeo, D., Shih, T.Y., Gonda, M.A., Young, H.A., Tsuchida, N., Lowy, D.R., Scolnick, E.M. (1981) Nature 292, 506-511.
30. Jelinek, W.R., Tooney, T.P., Leinwand, L., Duncan, C.H., Biro, P., Choudary, A., Weissman, P.V., Rubin, S.M., Houch, C.M. Deninger, P.L., Schmid, C.W. (1980) Proc. Nat. Acad. Sci. USA 77, 1398-1402.
31. Melton, D.A., Krieg, P.A., Rebagliati, M.R., Maniatis, T., Zinn, K., Green, M.R. (1984) Nuc. Acid Res. 12, 7035-7056.
32. Southern, E.M. (1975) J. Mol. Biol. 98, 503-517.
33. Maniatis, T., Jeffrey, A., Kleid, D.G. (1975) Proc. Nat. Acad. Sci. USA 72, 1184-1188.
34. Thomas, P.S. (1980) Proc. Nat. Acad. Sci. USA 77, 5501-5505.
35. Sharp, P.A., Gallimore, P.H., Flint, S.J (1974) Cold Spring Harbor Sympos. Quant. Biol. 39, 457-474.
36. Hohn, B., Murray, K. (1977) Proc. Nat. Acad. Sci. USA 74, 3259-3263.
37. Benton, W., Davis, R. (1977) Science 196, 180-182.
38. Gubler, U., Hoffman, B.J. (1983) Gene 25, 263-269.
39. Fasano, O., Birbaum, D., Edlund, L., Fogh, J., Wigler, M. (1984) Mol. Cell. Biol. 4:1695-1705.
40. Gimenez-Gallego, G., Rodkey, J., Bennett, C., Rios-Candelore, M., DiSalvo, J., Thomas, K. (1985) Science, 230, 1385-1388.
41. Abraham, J.A., Mergia, A., Whang, J.L., Tumolo, A., Friedman, J., Hjerrild, K.A., Gospodarowicz, D., Fiddes, J.C. (1986) Science, 233, 545-548.
42. Moore, R., Casey, G., Brookes, S., Dixon, M., Peters, G., Dickson, C. (1986) EMBO, 5, 919-924.
43. Taira, M., Yoshida, T., Miyagawa, K., Sakamoto, H. Terada, M., Sugimura, T. (1987) Proc. Nat. Acad. Sci. USA 84, 2980-2984.
44. Wigler, M., et al. (1979) Cell, 16, 777-785.
45. Zahn, X., Culpepper, A., Reddy, M., Loveless, J., Goldfarb, M. (1987), Oncogene 1: 369-376
46. J.C., Gospadarowicz, D. (187), Nature 325, 257-259.
47. Sanger, F., Nicklen, S., Coulson, A.R. (1977), Proc. Nat. Acad. Sci. USA 74, 5463-5467.
48. Reth, M., Alt, F. (1984), Nature 312, 418-423.
49. Pledger, W.J., Stiles, C.D., Antoniades, H.N., Scher, C.D. (1977), Proc. Nat. Acad. Sci. USA 74, 4481-4485.
50. Maciag, T., Mehlman, T., Freisel, R., Schreiber, A. (1984), Science 225, 932-935.
51. Shing, Y., Folkman, J., Sullivan, R.M Butterfield, C., Murray, J., Klagsburn, M. (1984), Science 223, 1296-1299.
52. Conn, G., Hatcher, V.B. (1984), Biochem. Biophys. Common. 124, 262-268.
53. Blobel, G., Walter, P., Change, G.N., Goldman, B.M., Erickson, A.H., Lingappa, V.R. (1979), Symp. Soc. Exp. Biol. 33, 9-36.
54. Yoshida, T., Miyagawa, K., Odagiri, H., Sakamoto, H., Little, P.F.R., Terada, M., Sugimura, T. (1987), Proc. Nat. Acad. Sci. USA 84, 7305-7309.
55. Moore, R., Casey, G., Brookes, S., Dixon, M., Peters, G., Dickson, C. (1986), EMBO J. 5, 919-924.
56. Abraham, J., Mergia, A., Whang, J.L., Tumulo, A., Friedman, J., Hjerrild, K.A., Gospadarowicz, D., Fiddes, J.C. (1986), Science 233, 545-548.
57. Tanese, et al., J. Virology (1986) 59: 328.

## Claims

**1.** A purified polypeptide having the amino acid sequence shown in Figure 5 for ORF-2.

**2.** A purified nucleic acid molecule encoding the polypeptide of claim 1.

**3.** A purified nucleic acid molecule of claim 2, wherein the purified nucleic acid molecule is DNA.

**4.** A purified nucleic acid molecule of claim 2, wherein the purified nucleic acid molecule is cDNA having the nucleotide sequence shown in Figure 5.

**5.** A vector which comprises the nucleic acid sequence encoded by the purified nucleic acid molecule of claim 2.

**6.** A vector of claim 5, wherein the vector is a plasmid.

**7.** A vector of claim 5, wherein the plasmid is the plasmid designated pLTR-122 deposited in an E. coli strain under ATCC No. 67413.

**8.** A vector of claim 5, wherein the vector is a virus.

**9.** A host vector system for producing a polypeptide having the amino acid sequence shown in Fig. 5 which comprises the vector of claim 6 in a suitable host.

**10.** A host vector system of claim 9, wherein the suitable host is an eucaryotic cell.

**11.** A host vector system of claim 10, wherein the eucaryotic cell is a mammalian cell.

**12.** A host vector system of claim 11, wherein the mammalian cell is a NIH 3T3 cell.

**13.** A method for producing a polypeptide having the amino acid sequence shown in Figure 5 comprising growing the host vector system of claim 9 so as to produce the polypeptide in the host and recovering the polypeptide so produced.

**14.** A pharmaceutical composition comprising an effective amount of the polypeptide, of claim 1, and a pharmaceutically acceptable carrier.

**15.** The pharmaceutical composition of claim 14 for stimulating the proliferation of mesodermal cells.

**16.** The pharmaceutical composition of claim 15, wherein the mesodermal cells are vascular endothelial cells.

**17.** The pharmaceutical composition of claim 14 for stimulating capillary growth.

**18.** A pharmaceutical composition of claim 14 for promoting tissue repair in a subject with damaged tissue.

**19.** An isolated fragment of total human genomic DNA comprising the oncogene encoding the amino acid sequence shown in Figure 5 for ORF-2.

**20.** A reagent comprising an antibody capable of specifically forming a complex with the polypeptide of claim 1.

**21.** A reagent of claim 20, wherein the antibody is a polyclonal antibody.

**22.** A reagent of claim 20, wherein the antibody is a monoclonal antibody.

**23.** A reagent of claim 20 for diagnosing in a subject a neoplastic condition associated with the presence of an activated oncogene.

**24.** A reagent of claim 20 for diagnosing in a human a neoplastic condition associated with the presence of an activated oncogene.

**25.** A method for detecting in a body fluid the presence of at least a portion of the polypeptide of claim 1, wherein the detecting comprises contacting the polypeptide with a reagent of claim 20 to form an antibody-polypeptide complex, and detecting the complex so formed.

**26.** A nucleic acid molecule useful as a probe for detecting the oncogene encoding the amino acid sequence shown in Figure 5 for ORF-2.

**27.** A nucleic acid molecule of claim 26, wherein the nucleic acid molecule is a polynucleotide.

**28.** A nucleic acid molecule of claim 26, wherein the nucleic acid molecule is an oligonucleotide.

**29.** A reagent of claim 20 for use in the treatment of cancer.

**30.** A purified polypeptide having the amino acid sequence shown in Figure 5 for ORF-1.

**31.** A purified nucleic acid molecule encoding the polypeptide of claim 30.

**Patentansprüche**

**1.** Gereinigtes Polypeptid mit der in Figur 5 für den offenen Leserahmen 2 (ORF-2) dargestellten Aminosäuresequenz.

**2.** Gereinigtes Nucleinsäuremolekül mit Kodierung für das Polypeptid nach Anspruch 1.

**3.** Gereinigtes Nucleinsäuremolekül nach Anspruch 2, wobei das gereinigte Nucleinsäuremolekül DNA ist.

**4.** Gereinigtes Nucleinsäuremolekül nach Anspruch 2, wobei das gereinigte Nucleinsäuremolekül cDNA mit der in Figur 5 dargestellten Nucleotidsequenz ist.

**5.** Vektor, umfassend die durch das gereinigte Nucleinsäuremolekül nach Anspruch 2 kodierte Nucleinsäuresequenz.

**6.** Vektor nach Anspruch 5, wobei es sich bei dem Vektor um ein Plasmid handelt.

**7.** Vektor nach Anspruch 5, wobei es sich bei dem Plasmid um das in einem E. coli-Stamm unter der ATCC-Nr. 67413 hinterlegte und mit pLTR-122 bezeichnete Plasmid handelt.

**8.** Vektor nach Anspruch 5, wobei es sich bei dem Vektor um ein Virus handelt.

**9.** Wirt/Vektor-System zur Herstellung eines Polypeptids mit der in Figur 5 dargestellten Aminosäuresequenz, umfassend den Vektor nach Anspruch 6 in einem geeigneten Wirt.

**10.** Wirt/Vektor-System nach Anspruch 9, wobei es sich bei dem geeigneten Wirt um eine eukaryontische Zelle handelt.

**11.** Wirt/Vektor-System nach Anspruch 10, wobei es sich bei der eukaryontischen Zelle um eine Säugetierzelle handelt.

**12.** Wirt/Vektor-System nach Anspruch 11, wobei es sich bei der Säugetierzelle um eine NIH 3T3-Zelle handelt.

**13.** Verfahren zur Herstellung eines Polypeptids mit der in Figur 5 dargestellten Aminosäuresequenz durch Wachsenlassen des Wirt/Vektor-Systems nach Anspruch 9 zur Produktion des Polypeptids in dem Wirt und Gewinnung des derart produzierten Polypeptids.

**14.** Arzneimittelzubereitung, umfassend eine wirksame Menge des Polypeptids nach Anspruch 1 und einen pharmazeutisch akzeptablen Träger.

**15.** Arzneimittelzubereitung nach Anspruch 14 zur Stimulierung der Proliferation mesodermaler Zellen.

**16.** Arzneimittelzubereitung nach Anspruch 15, wobei es sich bei den mesodermalen Zellen um Gefäßendothelzellen handelt.

**17.** Arzneimittelzubereitung nach Anspruch 14 zur Stimulierung des Kapillarwachstums.

**18.** Arzneimittelzubereitung nach Anspruch 14 zur Förderung einer Gewebereparatur bei einem Subjekt mit Gewebeschädigung.

**19.** Isoliertes Fragment der gesamten Humangenom-DNA, umfassend das Oncogen mit Kodierung für die in Figur 5 für ORF-2 dargestellte Aminosäuresequenz.

**20.** Reagens, umfassend einen Antikörper mit der Fähigkeit zur spezifischen Komplexbildung mit dem Polypeptid nach Anspruch 1.

**21.** Reagens nach Anspruch 20, wobei der Antikörper aus einem polyklonalen Antikörper besteht.

**22.** Reagens nach Anspruch 20, wobei der Antikörper aus einem monoklonalen Antikörper besteht.

**23.** Reagens nach Anspruch 20 zur Diagnose eines mit dem Auftreten eines aktivierten Oncogens verbundenen neoplastischen Zustands bei einem Subjekt.

**24.** Reagens nach Anspruch 20 zur Diagnose eines mit dem Auftreten eines aktivierten Oncogens verbundenen neoplastischen Zustands bei einem Menschen.

**25.** Verfahren zum Nachweis der Anwesenheit mindestens eines Teils des Polypeptids nach Anspruch 1 in einer Körperflüssigkeit, wobei der Nachweis das Inkontaktbringen des Polypeptids mit einem Reagens nach Anspruch 20 zur Bildung eines Antikörper/Polypeptid-Komplexes und den Nachweis des derart gebildeten Komplexes umfaßt.

**26.** Nucleinsäuremolekül mit der Eignung als Sonde zum Nachweis des Oncogens mit Kodierung für die in Figur 5 für ORF-2 dargestellte Aminosäuresequenz.

**27.** Nucleinsäuremolekül nach Anspruch 26, wobei es sich bei dem Nucleinsäuremolekül um ein Polynucleotid handelt.

**28.** Nucleinsäuremolekül nach Anspruch 26, wobei es sich bei dem Nucleinsäuremolekül um ein Oligonucleotid handelt.

**29.** Reagens nach Anspruch 20 zur Verwendung bei der Behandlung von Krebs.

**30.** Gereinigtes Polypeptid mit der in Figur 5 für ORF-1 dargestellten Aminosäuresequenz.

**31.** Gereinigtes Nucleinsäuremolekül mit Kodierung für das Polypeptid nach Anspruch 30.

**Revendications**

**1.** Polypeptide purifié présentant la séquence d'acides aminés indiquée sur la figure 5 pour ORF-2.

**2.** Molécule purifiée d'acide nucléique codant pour le polypeptide selon la revendication 1.

**3.** Molécule purifiée d'acide nucléique selon la revendication 2, dans laquelle la molécule purifiée d'acide nucléique est l'ADN.

**4.** Molécule purifiée d'acide nucléique selon la revendication 2, dans laquelle la molécule purifiée d'acide nucléique est l'ADNc présentant la séquence de nucléotides indiquée sur la figure 5.

**5.** Vecteur qui comprend la séquence d'acide nucléique codée par la molécule purifiée d'acide nucléique selon la revendication 2.

**6.** Vecteur selon la revendication 5, dans lequel le vecteur est un plasmide.

**7.** Vecteur selon la revendication 5, dans lequel le plasmide est le plasmide appelé pLTR-122 déposé dans une souche de E. coli sous ATCC n° 67413.

**8.** Vecteur selon la revendication 5, dans lequel le vecteur est un virus.

**9.** Système hôte de vecteur destiné à la production d'un polypeptide présentant la séquence d'acides aminés indiquée sur la figure 5, lequel comprend le vecteur selon la revendication 6 dans un hôte adapté.

**10.** Système hôte de vecteur selon la revendication 9, dans lequel l'hôte adapté est une cellule eucaryote.

**11.** Système hôte de vecteur selon la revendication 10, dans lequel la cellule eucaryote est une cellule de mammifère.

**12.** Système hôte de vecteur selon la revendication 11, dans lequel la cellule de mammifère est une cellule NIH 3T3.

**13.** Procédé de production d'un polypeptide présentant la séquence d'acides aminés indiquée sur la figure 5, lequel comprend le développement du système hôte de vecteur selon la revendication 9 de manière à produire le polypeptide dans l'hôte et la récupération du polypeptide ainsi produit.

**14.** Composition pharmaceutique comprenant une quantité efficace du polypeptide selon la revendication 1 et un véhicule pharmaceutiquement acceptable.

**15.** Composition pharmaceutique selon la revendication 14 destinée à la stimulation de la prolifération de cellules mésodermiques.

**16.** Composition pharmaceutique selon la revendication 15, dans laquelle les cellules mésodermiques sont des cellules endothéliales vasculaires.

**17.** Composition pharmaceutique selon la revendication 14 destinée à la stimulation du développement de capillaires.

**18.** Composition pharmaceutique selon la revendication 14 destinée à la favorisation de la réparation d'un tissu chez un sujet présentant un tissu lésé.

**19.** Fragment isolé d'ADN génomique total humain comprenant le gène oncogène codant pour la séquence d'acides aminés indiquée sur la figure 5 pour ORF-2.

**20.** Réactif comprenant un anticorps, capable de former spécifiquement un complexe avec le polypeptide selon la revendication 1.

**21.** Réactif selon la revendication 20, dans lequel l'anticorps est un anticorps polyclonal.

**22.** Réactif selon la revendication 20, dans lequel l'anticorps est un anticorps monoclonal.

**23.** Réactif selon la revendication 20 destiné au diagnostic chez un sujet d'un trouble néoplastique associé à la présence d'un gène oncogène activé.

**24.** Réactif selon la revendication 20 destiné au diagnostic chez un humain d'un trouble néoplastique associé à la présence d'un gène oncogène activé.

**25.** Procédé de détection dans un liquide organique de la présence d'au moins une fraction du polypeptide selon la revendication 1, dans lequel la détection comprend la mise en contact du polypeptide avec un réactif selon la revendication 20 pour former un complexe anticorps-polypeptide, et la détection du complexe ainsi formé.

**26.** Molécule d'acide nucléique utile en tant que sonde de détection du gène oncogène codant pour la séquence d'acides aminés indiquée sur la figure 5 pour ORF-2.

**27.** Molécule d'acide nucléique selon la revendication 26, dans laquelle la molécule d'acide nucléique est un polynucléotide.

**28.** Molécule d'acide nucléique selon la revendication 26, dans laquelle la molécule d'acide nucléique est un oligonucléotide.

**29.** Réactif selon la revendication 20 à utiliser dans le traitement du cancer.

**30.** Polypeptide purifié présentant la séquence d'acides aminés indiquée sur la figure 5 pour ORF-1.

**31.** Molécule purifiée d'acide nucléique codant pour le polypeptide selon la revendication 30.

Figure 1

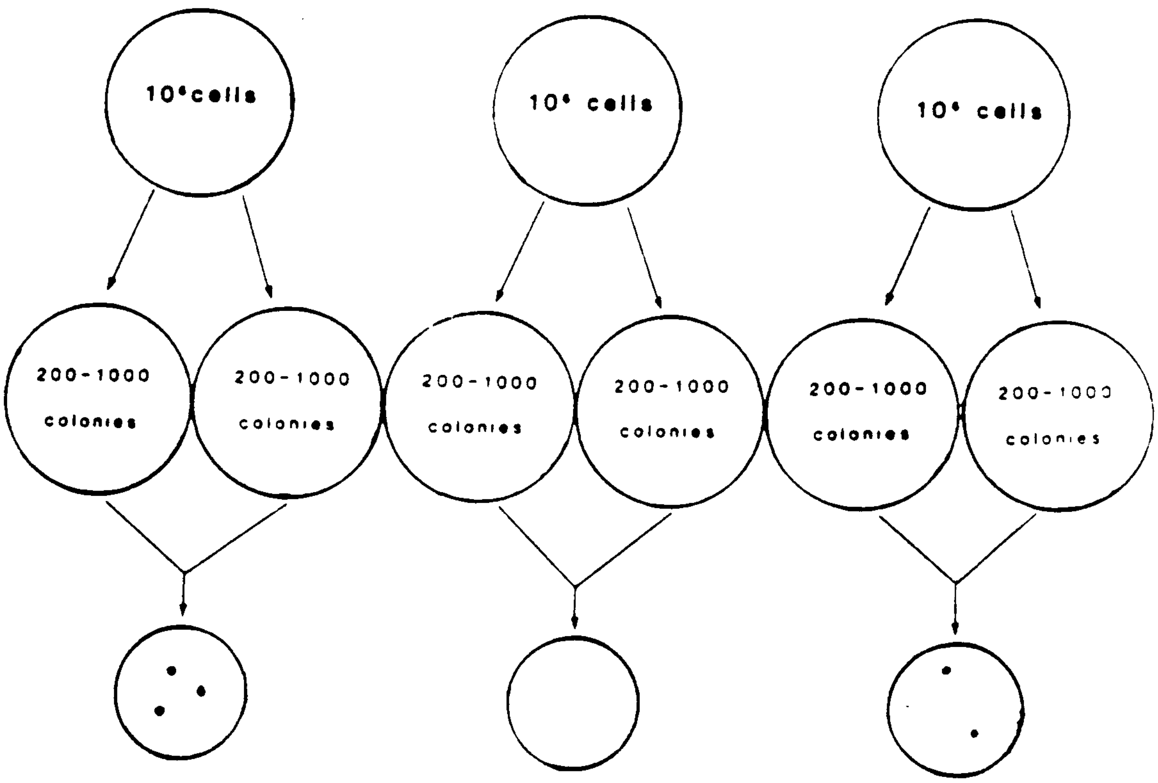
10⁶cells
10⁶ cells
10⁶ cells
200-1000
colonies
200-1000
colonies
200-1000
colonies
200-1000
colonies
200-1000
colonies
200-1000
colonies

Figure 2

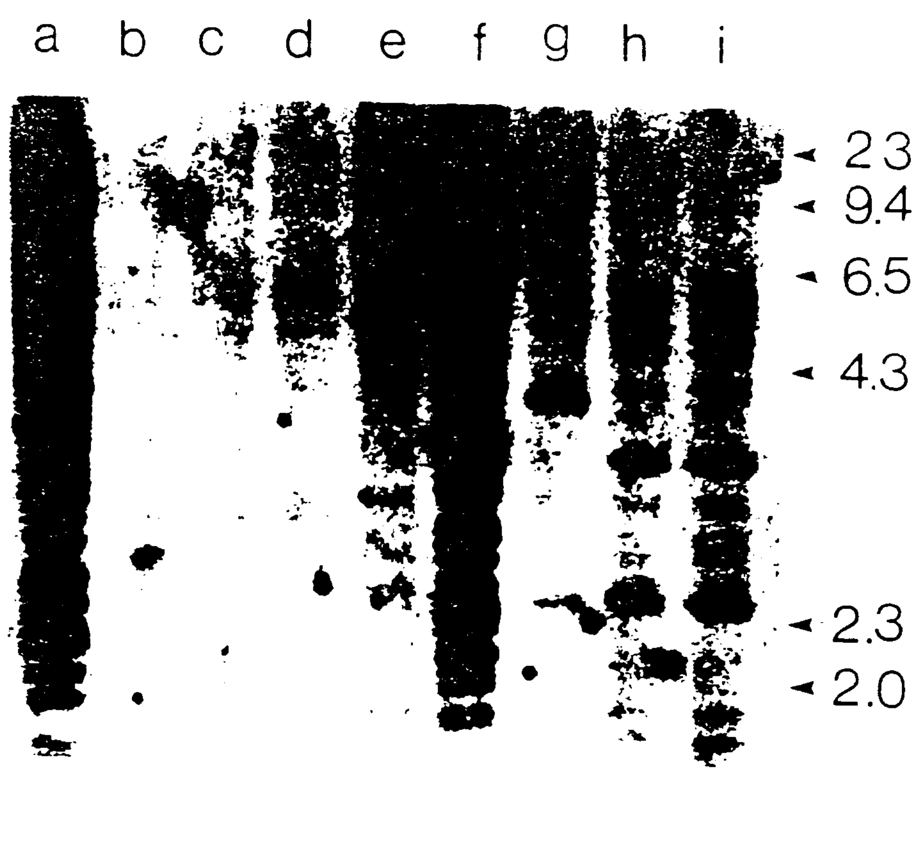

Figure 3

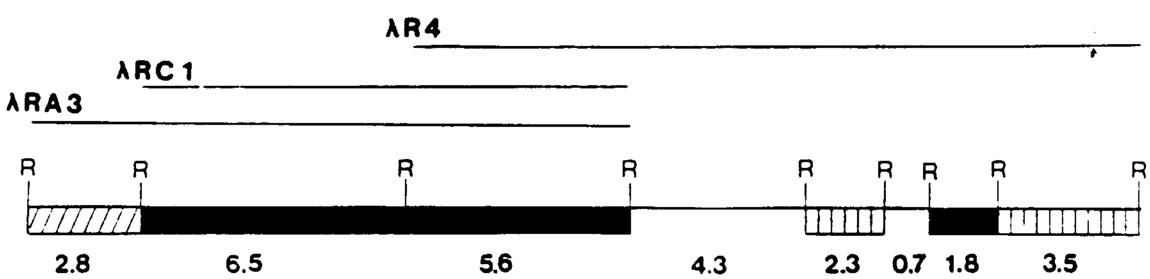

Figure 4

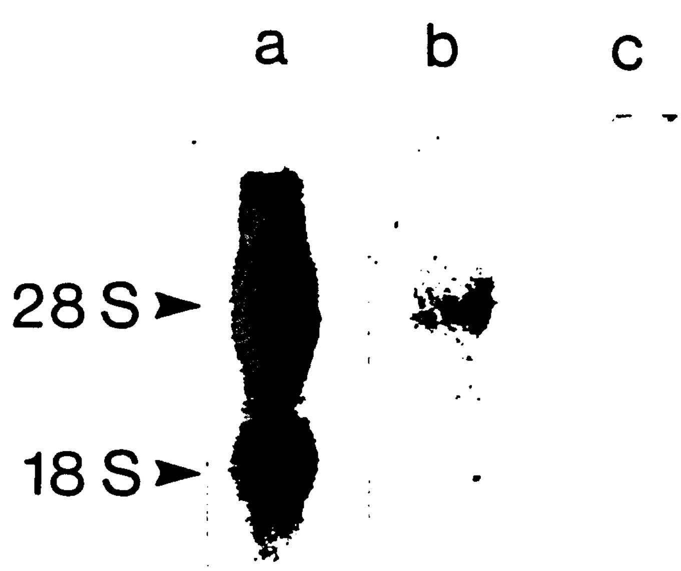

Figure 5

A)

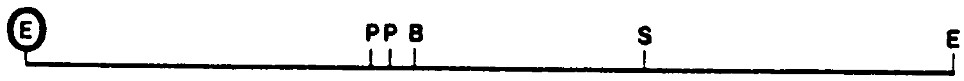


B)

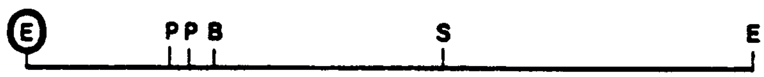


C)

ORF-1.....................METSerThrArgCysGlyGluAlaGlyGlnSerArgGlyThrGlnProHisArgGlyTyrArg 20
CCTCTCCCCTTCTCTTCCCCGAGGCTATGTCCACCCGGTGCGGCGAGGCGGGCCAGAGCAGAGGCACGCAGCCGCACAGGGGCTACAGA 89

AlaGlnAsnGlnProTyrLysMetHisLeuGlyProProArgLeuGluGluEND 38
GCCCAGAATCAGCCCTACAAGATGCACTTAGGACCCCCGCGGCTGGAAGAATGAGCTTGTCCTTCCTCCTCCTCCTCTTCTTCAGCCAC 178
ORF-2.........................................METSerLeuSerPheLeuLeuLeuLeuPhePheSerHis 13

CTGATCCTCAGCGCCTGGGCTCACGGGGAGAAGCGTCTCGCCCCCAAAGGGCAACCCGGACCCGCTGCCACTGATAGGAACCCTAGAGGC 268
LeuIleLeuSerAlaTrpAlaHisGlyGluLysArgLeuAlaProLysGlyGlnProGlyProAlaAlaThrAspArgAsnProArgGly 43

TCCAGCAGCAGACAGAGCAGCAGTAGCGCTATGTCTTCCTCTTCTGCCTCCTCCTCCCCCGCAGCTTCTCTGGGCAGCCAAGGAAGTGGC 358
SerSerSerArgGlnSerSerSerSerAlaMetSerSerSerSerAlaSerSerSerProAlaAlaSerLeuGlySerGlnGlySerGly 73

TTGGAGCAGAGCAGTTTCCAGTGGAGCCTCGGGGCGCGGACCGGCAGCCTCTACTGCAGAGTGGGCATCGGTTTCCATCTGCAGATCTAC 448
LeuGluGlnSerSerPheGlnTrpSerLeuGlyAlaArgThrGlySerLeuTyrCysArgValGlyIleGlyPheHisLeuGlnIleTyr 103

CCGGATGGCAAAGTCAATGGATCCCACGAAGCCAATATGTTAAGTGTTTTGGAAATATTTGCTGTGTCTCAGGGGATTGTAGGAATACGA 538
ProAspGlyLysValAsnGlySerHisGluAlaAsnMetLeuSerValLeuGluIlePheAlaValSerGlnGlyIleValGlyIleArg 133

GGAGTTTTCAGCAACAAATTTTTAGCGATGTCAAAAAAAGGAAAACTCCATGCAAGTGCCAAGTTCACAGATGACTGCAAGTTCAGGGAG 628
GlyValPheSerAsnLysPheLeuAlaMetSerLysLysGlyLysLeuHisAlaSerAlaLysPheThrAspAspCysLysPheArgGlu 163

CGTTTTCAAGAAAATAGCTATAATACCTATGCCTCAGCAATACATAGAACTGAAAAAACAGGGCGGGAGTGGTATGTTGCCCTGAATAAA 718
ArgPheGlnGluAsnSerTyrAsnThrTyrAlaSerAlaIleHisArgThrGluLysThrGlyArgGluTrpTyrValAlaLeuAsnLys 193

AGAGGAAAAGCCAAACGAGGGTGCAGCCCCCGGGTTAAACCCCAGCATATCTCTACCCATTTTCTTCCAAGATTCAAGCAGTCGGAGCAG 808
ArgGlyLysAlaLysArgGlyCysSerProArgValLysProGlnHisIleSerThrHisPheLeuProArgPheLysGlnSerGluGln 223

CCAGAACTTTCTTTCACGGTTACTGTTCCTGAAAAGAAAAATCCACCTAGCCCTATCAAGTCAAAGATTCCCCTTTCTGCACCTCGGAAA 898
ProGluLeuSerPheThrValThrValProGluLysLysAsnProProSerProIleLysSerLysIleProLeuSerAlaProArgLys 253

AATACCAACTCAGTGAAATACAGACTCAAGTTTCGCTTTGGATAATATTAATCTTGGCCTTGTGAGAAACCATTCTTTCCCCTCAGGAGT 988
AsnThrAsnSerValLysTyrArgLeuLysPheArgPheGlyEND 267

TTCTATAGGTGTCTTCAGAGTTCTGAAGAAAAATTACTGGACACAGCTTCAGCTATACTTACACTGTATTGAAGTCACGTCATTTGTTTC 1078

AGTGTGACTGAAACAAAATGTTTTTTGATAGGAAGGAAACTG 1120

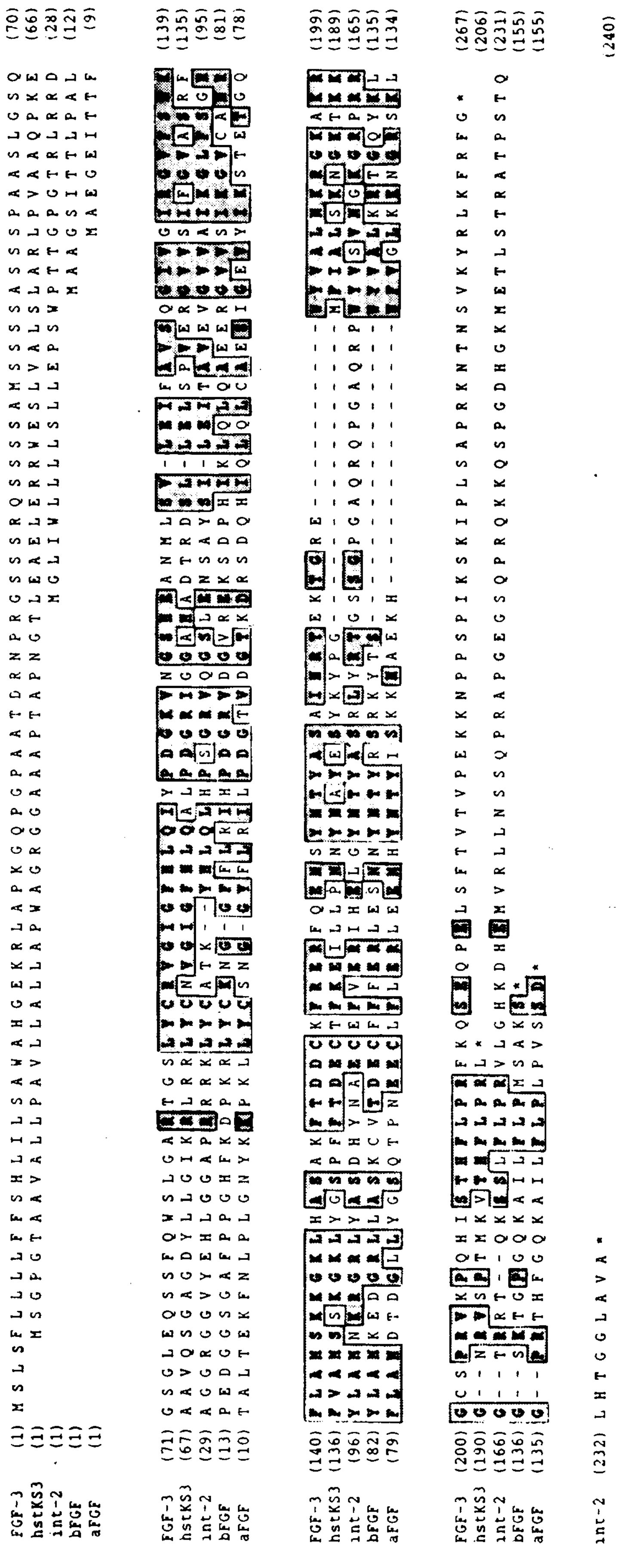

FIG. 6

Figure 7

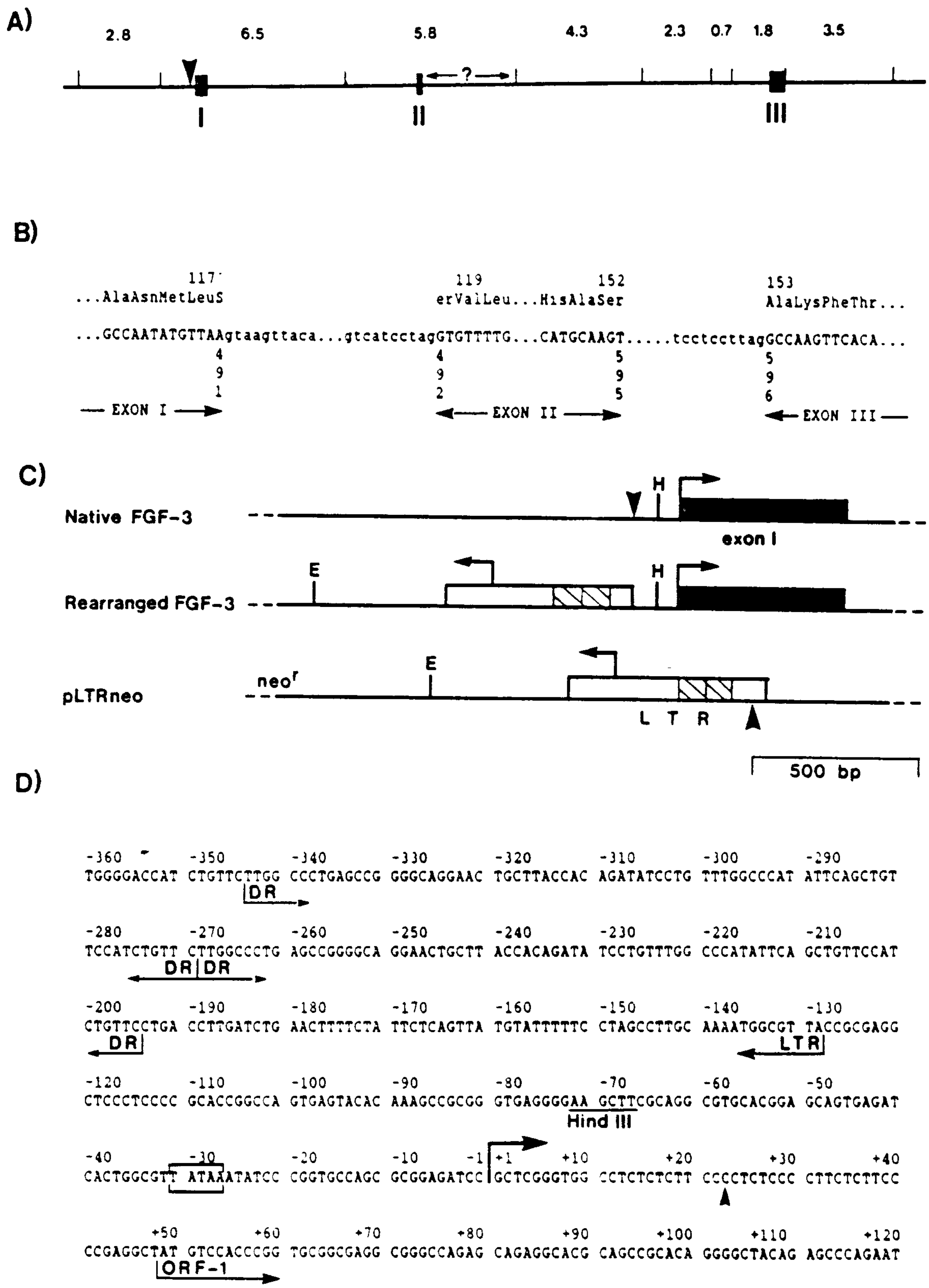
A)
2.8
6.5
5.8
4.3
2.3
0.7
1.8
3.5
?
I
II
III
B)
117
...AlaAsnMetLeuS
119
erValLeu...HisAlaSer
152
153
AlaLysPheThr...
...GCCAATATGTTAAgtaagttaca...gtcatcctagGTGTTTTG...CATGCAAGT.....tcctccttagGCCAAGTTCACA...
491
492
595
596
EXON I
EXON II
EXON III
C)
Native FGF-3
H
exon I
Rearranged FGF-3
E
H
pLTRneo
neo^r
E
L T R
500 bp
D)
-360 -350 -340 -330 -320 -310 -300 -290
TGGGGACCAT CTGTTCTTGG CCCTGAGCCG GGGCAGGAAC TGCTTACCAC AGATATCCTG TTTGGCCCAT ATTCAGCTGT
DR
-280 -270 -260 -250 -240 -230 -220 -210
TCCATCTGTT CTTGGCCCTG AGCCGGGGCA GGAACTGCTT ACCACAGATA TCCTGTTTGG CCCATATTCA GCTGTTCCAT
DR DR
-200 -190 -180 -170 -160 -150 -140 -130
CTGTTCCTGA CCTTGATCTG AACTTTTCTA TTCTCAGTTA TGTATTTTTC CTAGCCTTGC AAAATGGCGT TACCGCGAGG
DR
LTR
-120 -110 -100 -90 -80 -70 -60 -50
CTCCCTCCCC GCACCGGCCA GTGAGTACAC AAAGCCGCGG GTGAGGGGAA GCTTCGCAGG CGTGCACGGA GCAGTGAGAT
Hind III
-40 -30 -20 -10 -1 +1 +10 +20 +30 +40
CACTGGCGTT ATAAATATCC CGGTGCCAGC GCGGAGATCC GCTCGGGTGG CCTCTCTCTT CCCCTCTCCC CTTCTCTTCC
+50 +60 +70 +80 +90 +100 +110 +120
CCGAGGCTAT GTCCACCCGG TGCGGCGAGG CGGGCCAGAG CAGAGGCACG CAGCCGCACA GGGGCTACAG AGCCCAGAAT
ORF-1

Figure 8

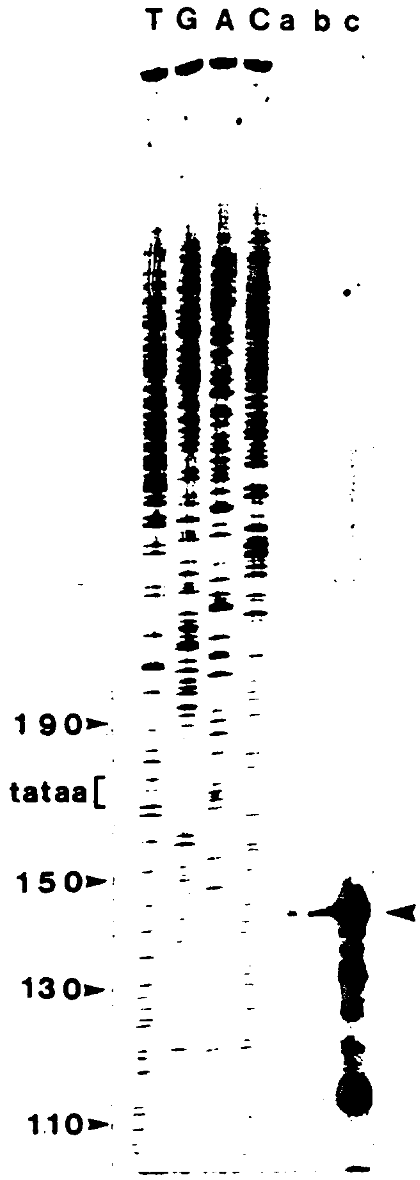

Figure 9 and 10

Fig. 9

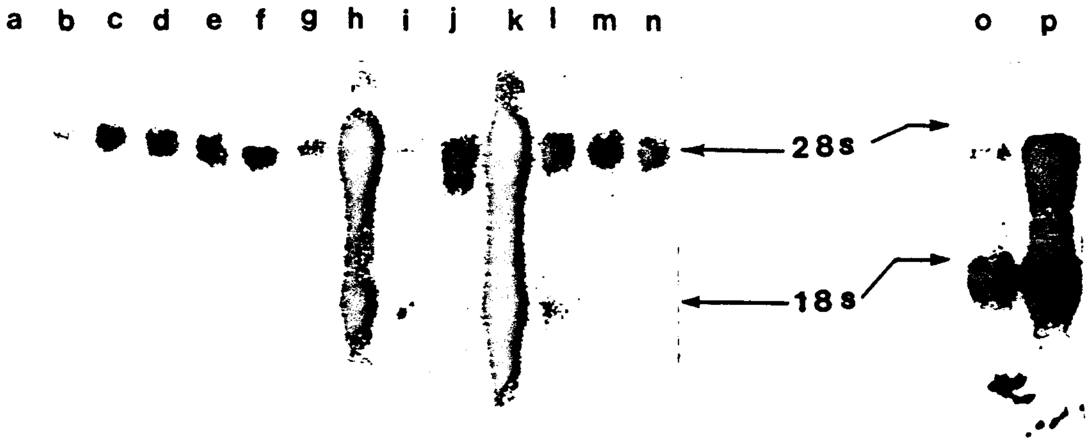


Fig. 10